# EUROPÄISCHE PATENTANMELDUNG

(11) **EP 2 455 757 A1**
(43) Veröffentlichungstag der Anmeldung: **23.05.2012**
(21) Anmeldenummer: 10191544.5
(22) Anmeldetag: 17.11.2010
(51) Int. Cl.: G01N 33/58

(54) **Verfahren zur Verstärkung von Signalen in heterogenen Bindungsassays**

(71) Anmelder: FZMB GmbH Forschungszentrum für Medizintechnik und Biotechnologie, 99947 Bad Langensalza (DE)
(72) Erfinder: Miethe, Peter, 99947 Bad Langensalza (DE); Pietraszczyk, Marko, 99427 Weimar (DE)
(74) Vertreter: von Kreisler Selting Werner

(57) **Zusammenfassung**

Verfahren zur Verstärkung von Signalen in heterogenen Bindungsassays auf Basis eines Donator/Rezeptorkomplexes 1 aus einem Rezeptormolekül (10) und einem Donatormolekül (20), das vom Rezeptormolekül (10) gebunden wird wobei
- in einem ersten Schritt der Donator/Rezeptorkomplex 1, gebildet wird,
- danach in einem zweiten Schritt mindestens ein Partikel einer ersten Partikelklasse zu dem gebildeten Donator/Rezeptorkomplex 1 gegeben wird,
- der mindestens eine Partikel der ersten Partikelklasse an den Donator/Rezeptorkomplex 1 koppelt und
- der mindestens eine an den Donator/Rezeptorkomplex 1 gekoppelte Partikel der ersten Partikelklasse n ≥ 2, wobei n eine natürliche Zahl ist, weitere Bindungsstelle aufweist, an die mindestens ein Partikel einer zweiten Partikelklasse koppeln kann,
- der Partikel der zweiten Partikelklasse seinerseits mindestens m ≥ 2, wobei m eine natürliche Zahl ist, Bindungsstellen aufweist an die mindestens ein Partikel der ersten Partikelklasse koppeln kann,
- in einem dritten Schritt mindestens ein Partikel der zweiten Partikelklasse zugegeben wird und
gegebenenfalls die Schritte, insbesondere der zweite und dritte Schritt, mindestens einmal wiederholt werden.

## Beschreibung

Die vorliegende Erfindung betrifft ein Verfahren zur Verstärkung von Signalen in heterogenen Bindungsassays auf Basis von Donator/Rezeptorkomplexen, insbesondere Ligand - Rezeptorkomplexen.

Heterogene Bindungsassays sind in Form von Enzyme Linked Immuno Sorbent Assays (ELISA) sehr weit verbreitet. Bei ihnen werden Konjugate bestehend aus einem Detektionsantikörper und einem Enzym, vorzugsweise Meerettichperoxididase oder alkalische Phosphatase eingesetzt um nach Zugabe eines Substrates ein kolorimetrisch, fluorimetrisch oder luminometrisch gut detektierbares Signal zu erhalten. Der Hauptvorteil dieser Technik ist die gute Sensitivität die beispielsweise bei Proteinen Nachweisgrenzen im Bereich von einigen pg/ml zulässt. Ihr Hauptnachteil ist die Störanfälligkeit, insbesondere die starke Temperaturabhängigkeit der Enzymreaktion und die Notwendigkeit die benötigten Reagenzien (Konjugate, Substrate) bei 4 °C zu lagern. Diese Nachteile können umgangen werden, wenn an Stelle des Enzymkonjugates ein Farbstoffkonjugat eingesetzt wird. Insbesondere in Form von Goldpartikel-Antikörper oder Farbpartikel- Antikörper Konjugaten lassen sich damit einfache und gut zu lagernde heterogene Testsysteme herstellen. Zu den bekanntesten Bauformen können die in US-A-6,485,982 offenbarten lateral flow Teststreifen oder Testsäulen WO-A-92/05442, WO-A-94/09365 oder WO-A-95/19569 gezählt werden. Ein Nachteil dieser Formate ist ihre geringe Sensitivität, die bei Proteinen nur Nachweisgrenzen im Bereich von ca. 1 ng/ml zulässt.

Der Erfindung liegt die Aufgabe zu Grunde, ein Verfahren anzugeben, mit dem es gelingt die Empfindlichkeit der Analytik mit heterogenen Assays zu erhöhen, ohne eine enzymatische Verstärkungsreaktion einsetzen zu müssen.

Gelöst wird diese Aufgabe gemäß der Erfindung durch ein Verfahren zur Verstärkung von Signalen in heterogenen Bindungsassays auf Basis von Donator/Rezeptorkomplexen, wobei
- in einem ersten Schritt ein Donator/Rezeptorkomplex 1, gebildet wird,
- danach in einem zweiten Schritt mindestens ein Partikel einer ersten Partikelklasse zu dem gebildeten Donator/Rezeptorkomplex 1 gegeben wird,
- der mindestens eine Partikel der ersten Partikelklasse an den Donator/Rezeptorkomplex 1 koppelt und
- der mindestens eine an den Donator/Rezeptorkomplex 1 gekoppelte Partikel der ersten Partikelklasse n ≥ 2, wobei n eine natürliche Zahl ist, weitere Bindungsstelle aufweist, an die mindestens ein Partikel einer zweiten Partikelklasse koppeln kann,
- der Partikel der zweiten Partikelklasse seinerseits mindestens m ≥ 2, wobei m eine natürliche Zahl ist, Bindungsstellen aufweist an die mindestens ein Partikel der ersten Partikelklasse koppeln kann,
- in einem dritten Schritt mindestens ein Partikel der zweiten Partikelklasse zugegeben wird und
- gegebenenfalls die Schritte, insbesondere der zweite und dritte Schritt, mindestens einmal wiederholt werden.

Erfindungsgemäß wird unter dem Begriff Donator eine chemische Entität, insbesondere ein Molekül oder Molekülverband verstanden, der mit einem Rezeptor, der ebenfalls eine chemische Entität, insbesondere ein Molekül oder Molekülverband ist, eine starke Wechselwirkung ausübt. Diese Wechselwirkung führt zu der Ausbildung einer Koppelung zwischen Donator und Rezeptor. Die Kopplung ist eine spezifische Wechselwirkung deren Spezifität insbesondere durch die zur Charakterisierung von chemischen Komplexen verwendeten Größen erfassbar ist. Dies ist beispielsweise die Dissoziationskonstante K_{D} (reziprok zur Assoziationskonstanten), die sich aus dem Massenwirkungsgesetz ergibt. Generell gilt, je kleiner K_{D}, desto stärker ist die Bindung zwischen den den Komplex bildenden Komponenten Donator und Rezeptor. Typische Dissoziationskonstanten, die bei Bindung von Antikörpern vom IgG-Typ auftreten, liegen im Bereich von 10⁻⁴-10⁻¹¹ M, für das Donator-Rezeptor-Paar Biotin/Avidin liegt die Wechselwirkung im Bereich von 10⁻¹⁴ und 10⁻¹⁵ M. Diese kann z. B. durch elektrostatische Wechselwirkungen oder solche die durch Polarisierungen (Wasserstoffbrückenbindungen) entstehen, aber auch durch sogenannte van der Waals Attraktionen vermittelt werden. Oftmals werden diese Wechselwirkungen auch durch komplementäre Strukturelemente an der Oberfläche der jeweiligen Moleküle oder Molekülverbände ermöglicht. Dabei kann, je nach Betrachtungsweise, auch der Donator ein Rezeptor des Rezeptormoleküls sein. Dies wir deutlich beim Vergleich der Donator/Rezeptorpaare Avidin/Biotin, bei dem jeweils einer der Partner als Donator für den anderen, dann den Rezeptor darstellenden Part fungiert. Üblicherweise wird ein niedrigmolekularer Donator in Komplexen mit sehr großen Molekülen, beispielsweise Enzymen oder Neurorezeptoren, als Ligand bezeichnet.

Das erfindungsgemäße Verfahren nutzt insbesondere einen Immunkomplex als Donator/Rezeptorkomplex 1. Darunter wird ein Komplex aus einem Liganden, der ein Hapten oder Antigen sein kann und einem Molekül, das in der Immunreaktion eines Organismus mit Immunsystem bei der Erkennung von molekularen Strukturen, insbesondere Epitopen eine Rolle spielt, zum Beispiel Antikörper unabhängig von ihrer Klasse, oder Fragment dieser Antikörper, verstanden. In einer Ausführungsform des erfindungsgemäßen Verfahrens kann der Zugabe des mindestens einen Partikels der zweiten Partikelklasse im dritten Schritt mindestens ein Partikel einer dritten, von den Partikeln der ersten Partikelklasse unterschiedlichen Partikelklasse zugegeben werden. Dies weist den Vorteil auf, dass eine Kompetition der Partikel der ersten Partikelklassen miteinander ausgeschlossen ist.

Die Partikel weisen zumindest eine Bindungsstelle auf, die mit einer anderen Bindungsstelle einer anderen Partikelklasse einen Donator-Rezeptor-Komplex ausbilden kann. Dazu werden die Partikel, sofern sie nicht bereits von Natur aus solche Bindungsstellen aufweisen, modifiziert durch chemische Reaktionen, die die entsprechenden Donator- oder Rezeptormoleküle an der Oberfläche der Partikel der unterschiedlichen Partikelklasse binden. So kann beispielsweise an der Oberfläche der Partikelklasse 1 Avidin angeordnet sein, das dann mit dem Immunkomplex bindet, sofern dieser ein biotinylierter ist, wohingegen dann die Partikel der Partikelklasse 2 wiederum mit Biotinmolekülen an ihrer Oberfläche versehen sind, die dann an die Partikel der Partikelklasse 1 binden können.

Methoden, um die entsprechenden Donator- und Rezeptoreinheiten an den Partikeln der jeweiligen Partikelklasse zu binden, hängen von der Natur des Partikels und der Natur der Donator/Rezeptormoleküle, die zu koppeln sind, ab. Dem Fachmann steht dazu ein Arsenal von verschiedenen Möglichkeiten zur Verfügung, um jeweils für das entsprechende Problem geeignete Lösungen zu finden. Sofern die chemische Natur der Donator/Rezeptormoleküle dies zulässt, können diese bereits direkt an der Oberfläche der Partikel gekoppelt werden, es besteht aber auch die Möglichkeit, diese über sogenannte Spacer an der Oberfläche der Partikel zu binden. Sind die Partikel chemisch mehr oder weniger inert, kann in vorgelagerten Schritten die Oberfläche der Partikel chemisch aufbereitet werden, um Bindungen mit den insbesondere organischen Molekülen des Typs der Donatoren/Rezeptoren zu ermöglichen oder man kann die chemisch inerten Partikel mit modifizierbaren Beschichtungen versehen.

Erfindungsgemäß können in einer Ausführungsform n und m jeweils > 2, insbesondere 3- 10 sein. Bei n, m = 2 bildet sich eine lineare Verstärkung und bei n, m = 3 bereits eine flächige Verstärkung aus.

In einer anderen Ausführungsform der Erfindung weisen die Partikel der ersten und zweiten Partikelklasse ungefähr die gleiche Größe oder unterschiedliche Größe auf. Typischerweise können Nanopartikel oder Biopolymere verwendet werden. Die Partikelgröße ist von der jeweils verwendeten Sorte und/oder von dem jeweiligen Assay zugrunde liegenden Bedingungen abhängig und kann vom Fachmann mittels seiner durchschnittlichen Kenntnisse angepasst und gewählt werden. Typischerweise können die Partikel eine mittlere Größe von 5 nm bis 5 µm aufweisen, wobei dabei eine annähernd sphärische Form angenommen wird. Sollten die Partikel eine sehr unregelmäßige Form aufweisen sind diese Werte als deren größte Ausdehnung in einer Raumrichtung anzusehen. Erfindungsgemäß können als Partikel der Partikelklasse organische oder anorganische Partikel oder Kombinationen davon eingesetzt werden.

Als anorganische Partikel können im erfindungsgemäßen Verfahrens insbesondere Goldpartikel, magnetische Partikel, oder als organische Partikel, Partikel aus Polymeren und Biopolymeren, z.B. Latex, Proteine, insbesondere fluoreszierende Proteine wie Phycoerythrin, Phycocyanin, oder Zellulosederivate, Dextranderivate eingesetzt werden.

Typische Partikel sind Goldpartikel mit mittleren Durchmessern von 5-50 nm, insbesondere 10-20 nm, Latexpartikel mit Farbstoff oder Fluoreszenzfarbstoff in einer Polymermatrix mit mittleren Durchmessern von 10 nm-4 µm, insbesondere 100 nm-400 nm), Farbstoffdispersionen mit Partikeln mit mittleren Durchmessern von 100 nm-500 nm, wie sie in WO-A-97/19354 offenbart sind, Magnetpartikel mit mittleren Durchmessern in einer Größe von 10 nm-4 µm, insbesondere 40 nm-400 nm oder Polymere, insbesondere Biopolymere wie Proteine insbesondere fluoreszierende Proteine wie Phycoerythrin, Phycocyanin, oder Zellulosederivate, Dextranderivate.

In einer weiteren Ausführungsform des erfindungsgemäßen Verfahrens sind die Partikel der ersten, zweiten und/oder dritten Partikelklasse detektierbar.

Die freien Bindungsstellen der ersten und zweiten Partikelklassen können in einer Ausführungsform des erfindungsgemäßen Verfahrens wechselseitig durch ein Ligand/Rezeptorpaar als Donator/Rezeptorpaar gebildet werden. So kommen beispielsweise als wechselseitige Ligand/Rezeptorpaare Avidin/Streptavin, Protein A/ IgG, insbesondere der F_{c} Teil von IgG, Lektin/Oligosaccarid, Polymerbindungsdomaine/ Biopolymer, anti Hapten-Antikörper/Hapten oder Kombinationen davon in Betracht.

Die Detektion der detektierbaren Partikel erfolgt insbesondere durch optische Methoden wie Durchlichtphotometrie, Reflektometrie, Fluorimetrie, Luminometrie, Bestimmung der Brechzahl und Doppelbrechung, durch bildgebende Verfahren, durch elektrische Methoden wie Konduktometrie, Potentiometrie, oder magnetische Methoden wie lineare und nichtlineare Suszeptometrie und Relaxometrie auf Basis von Giant Magnetic Resistoren, Hallsensoren, Flux Gate Sensoren, Squids oder Induktionsspulen.
Figur 1A zeigt schematisch die Ausbildung eines Immunkomplexes aus auf einer Festphase immobilisiertem Antikörper und einem Liganden.
Figur 1B zeigt schematisch die Bindung eines Partikels der Klasse 1 an den Immunkomplex. Der Partikel der Klasse 1 besitzt Spezifitäten gegen den Analyten (n1 = 4) sowie gegen Partikel der Partikelklasse 2 (n2 = 4).
Figur 1 C zeigt schematisch die Situation, die vorliegt, wenn Partikel der Klasse 2 (m = 8) an das Aggregat gemäß Fig. 1B gebunden haben. Figur 1D zeigt schematisch die Situation nach mehrmaligem Durchlaufen des erfindungsgemäßen Verfahrens.
Die Figuren 2 A - D zeigen die Ausbildung der Verstärkung, die gemäß Figur 1 allgemein beschrieben wurde für eine Ausführungsform, bei der Avidin an den Partikeln der Klasse 1 und Biotin an den Partikeln der Klasse 2 immobilisiert sind.
Die Figuren 3 A - D zeigen eine zu der Ausführungsform gemäß Figuren 2 A - 2D analoge Durchführung der Verstärkungsreaktion, wobei die Partikel der Klasse 2 als Proteinmoleküle 52 (hier BSA) ausgeführt sind.
Die Figuren 4 A - D zeigen die Ausbildung der Verstärkung, die gemäß Figur 1 allgemein beschrieben, wurde für eine Ausführungsform, bei der Antikörper gegen FITC und Antikörper gegen den zu bestimmenden Analyten an den Partikeln der Klasse 1 und FITC an den Partikeln der Klasse 2 immobilisiert sind.

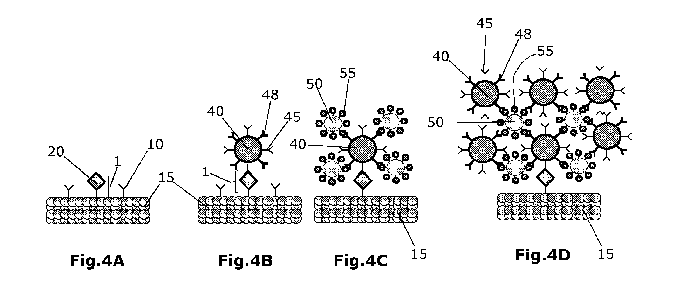

Die Figuren 4 A - D zeigen die Ausbildung der Verstärkung, in einem indirekten serologischen Assay zum Nachweis von IgG für eine Ausführungsform, in der drei Partikelklassen zum Einsatz gelangen. Dabei sind Antikörper gegen die F_{c}-Region eines Antikörpers der den Analyten bindet und Biotin an den Partikeln der Klasse 1, Avidin an Partikeln der Klasse 2 und Biotin an den Partikeln der Klasse 3 immobilisiert.

### Detaillierte Beschreibung der Erfindung

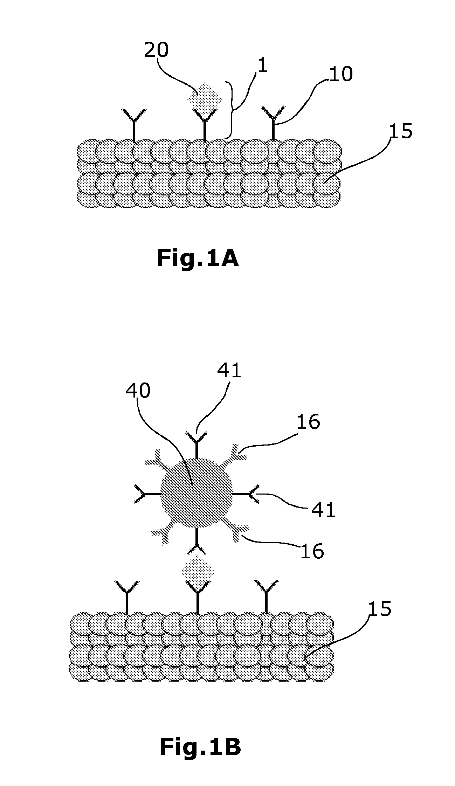

Figur 1 A-D zeigt in schematisierter Form eine Ausführungsform der vorliegenden Erfindung. Das damit verbundene Prinzip beleuchtet den Aufbau des erfindungsgemäßen Verfahrens und dessen Ergebnis. Auf einem Träger 15 sind Rezeptormoleküle 10 immobilisiert, die mit einem Donatormolekül 20 einen Donator-/Rezeptorkomplex 1 ausbilden können. Dieser Rezeptor/Donatorkomplex 1 besteht aus dem Rezeptor 10 und Donator 20. Danach wird in einem zweiten Schritt ein Partikel 40 an dessen Oberfläche ebenfalls Rezeptormoleküle 41 immobilisiert sind, die mit dem Donator 20 eine Bindung eingehen können, versetzt. Die Partikel 40 der Partikelklasse 1 binden mithin an den Donator-/Rezeportkomplex. Die Partikel 40 der Partikelklasse 1 weisen neben den Rezeptormolekülen 10 Gruppen 16 auf, die mit komplementären Gruppen 18 auf Partikeln 50 der Partikelklasse 2 in Wechselwirkung treten können. Die auf dem Partikel 40 der Partikelklasse 1 angeordneten zusätzlichen Rezeptormoleküle 10, die mit den komplementären am Partikel 50 der Klasse 2 immobilisierten Molekülen, quasi Donatorenmoleküle 20, in Wechselwirkung treten können, sorgen für eine dauerhafte Wechselwirkung zwischen den Partikeln 40 der Klasse 1 und den Partikeln 50 der Klasse 2. Danach sorgt die weitere Zugabe von Partikeln 40 der Klasse 1, die mit freien Donatoren Molekülen 18 auf den Partikeln 50 der Partikelklasse 2 wechselwirken für eine weitere Verstärkung des gebildeten Multipartikelkomplexes. Dabei wirken die Moleküle 18 der Partikelklasse 2 praktisch als Rezeptoren für die im vorigen Schritt noch als Rezeptoren auf den Partikeln 40 der Partikelklasse 2 bezeichneten, in diesem Schritt allerdings als Donatoren 20 wirkenden Molekülen.

Insgesamt lässt sich durch diese Methodik eine Verstärkung eines Signals erzielen, wenn entsprechend markierte Partikel eingesetzt werden. Die Markierung kann dann durch geeignete Detektionsmethoden ausgelesen werden.

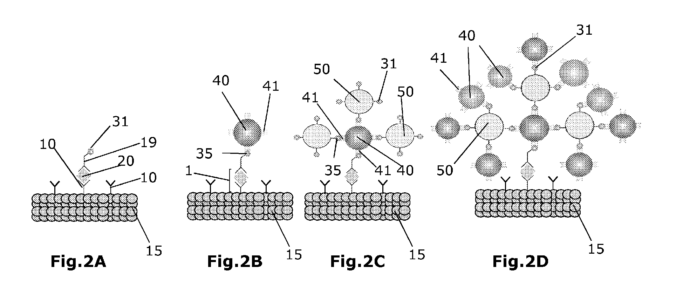

Varianten des erfindungsgemäßen Verfahrens können in den Figuren 2-4 verfolgt werden. Der Einfachheit halber wird das Prinzip anhand der Bildung von Immunkomplexen 1 näher erläutert. Dabei kann der Fachmann einfacherweise durch die Ersetzung des Begriffes "Immunkomplex" durch "Donator-/Rezeptorkomplex", die hinter dem Assay gemäß vorliegender Erfindung steckenden allgemeinen Prinzips ohne weiteres erkennen und abstrahieren.

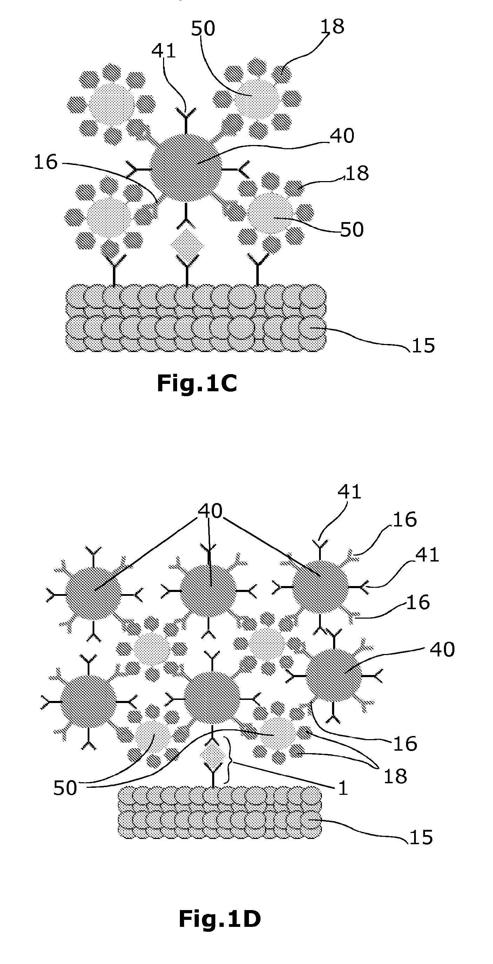

Zunächst wird daher am Beispiel der Figur 1 das System anhand der Konkretisierung eines Immunkomplexes näher erläutert.

Die Fig. 1A zeigt einen typischen Immunkomplex 1, bei dem ein Fängerantikörper 10 ein Antigen 20 gebunden hat. Der Fängerantikörper 10 ist an einem Träger 15 immobilisiert. Dieser Komplex 1 weist, präsentiert durch das Antigen 20, eine Bindungsstelle für Partikel 40 der Klasse 1 auf.

Im zweiten Schritt entsprechend Fig. 1B binden Partikel 40 der Klasse 1 über ihre Antigenspezifität 41 an den Immunkomplex 1. Darüber hinaus besitzen die Partikel 40 der Klasse 1 eine Spezifität 16 gegen Partikel 50 der Klasse 2. In einem dritten Schritt entsprechend Fig. 1C binden Partikel 50 der Klasse 2 an Partikel 40 der Klasse 1, durch Wechselwirkung der Spezifität 16 des Partikels 40 mit der Spezifität 18 des Partikels 50. In Abhängigkeit von Stöchiometrie und räumlichen Ausbildung der Bindungspartner entsteht ein Multipartikelkomplex. Durch wechselseitige Zugabe von Markierungen, insbesondere farbige Partikel der Klasse 1 und farbige Partikel der Klasse 2, wird der Komplex 1 entsprechend vergrößert (Siehe Fig 1D).

Der Vorgang kann mehrfach auch unter Einbeziehung von Waschschritten fortgeführt werden. Bei Einsatz von Farbpartikeln ist nach jedem Zugabeschritt eine Erhöhung der Farbintensität zu verzeichnen. Die Nachweismethode ist besonders einfach und vorteilhaft durchzuführen, wenn zur Immobilisierung des Fängerantikörpers 10 dreidimensionale poröse Träger 15 wie Filter oder Sintermaterialien eingesetzt werden und die einzelnen Schritte im Durchfluss in einer Säule oder auf einer Lateralflussmembran (lateral flow membran) durchgeführt werden. Typische Verweil- bzw. Inkubationszeiten für die einzelnen Schritte liegen im Bereich von 10 sec bis 10 min (vorzugsweise 30 s bis 2 min).

In der Variante gemäß Figuren 2A - 2D werden die Schritte, die in Figur 1A - 1D bereits beschrieben worden waren variiert. Auch hier wird zunächst ein Fängerantikörper 10, der auf einen Träger 15 immobilisiert ist, mit einer analytenthaltenden Probe in Kontakt gebracht, worauf sich ein Immunkomplex 1 zwischen dem Fängerantikörper 10 und dem Analyten 20 ausbildet. In einem darauf folgenden Schritt wird der Mischung ein Vermittler, in diesem Fall ein Detektorantikörper 30, der mit Biotin 31 versehen ist, zugefügt.

An den gebildeten Immunkomplex 1 - bestehend aus immobilisiertem Fängerantikörper 10, Analyt 20, Detektorantikörper 30 mit Biotin 31 - bindet dann ein Partikel 40 der Klasse 1, welcher mit Avidin 41 versehen ist.

Vorteilhaft ist in dieser Ausführungsform, dass der Partikel der Partikelklasse 1 und der Partikel der Partikelklasse 2 jeweils nur eine Spezifität aufweisen muss, um die Verstärkung hervorzurufen, da der Partikel der Klasse 1 über einen Vermittler, in diesem Fall ein biotinylierter Antikörper 19, an dem Immunkomplex bindet. Generell muss der Mediator ein Molekül sein, das zumindest zwei unterschiedliche Bindungsstellen, zum einen zum Analyten 20 und zum anderen zum Partikel 40 der Klasse 1, aufweist.

Figur 2C zeigt die Situation nach Zugabe eines Partikels 50 der Partikelklasse 2, welcher seinerseits mit Biotin 31 versehen ist. Aufgrund der Biotinmarkierung 31 des Partikel 50 der Partikelklasse 2 bindet der Partikel 50 an die bereits vorhandenen Partikel 40 der Klasse 1, die mit dem komplementären Avidin 41 versehen sind, was dann bereits zu einer ersten Verstärkung des auf dem Träger immobilisierten Signals führt.

Figur 2D zeigt nun in einem weiteren Schritt die Ausbildung eines noch komplexeren Systems, das nach Wiederholung des ersten Schrittes in dem Partikel 40 der Partikelklasse 1, die wiederum mit Avidin 41 markiert sind, entsteht. Die erneute Zugabe der Partikel 40 der ersten Partikelklasse, die mit Avidin 41 markiert sind, führt nun zu einer weiteren Verstärkung des Signals, indem diese Partikel 40 der Partikelklasse 1 an freie Biotinbindungsstellen 31 der im vorigen Schritt gemäß Figur 2C zugegebenen Partikel 50 der Partikelklasse 2 binden. Im Endeffekt ergibt sich eine durch eine Amplifikationsreaktion hervorgerufene Verstärkung des Signals und damit Erhöhung der Detektionsempfindlichkeit.

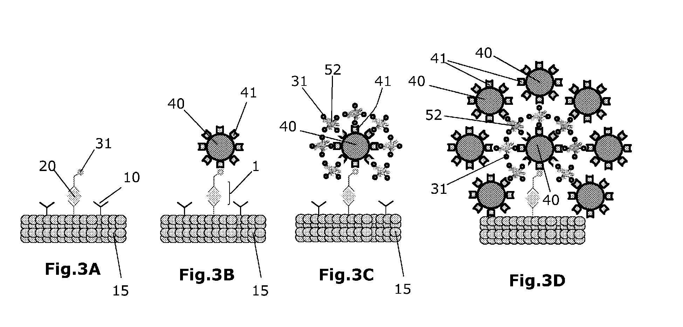

Die Figuren 3A - 3D veranschaulichen eine Variante der Ausführungsform gemäß Figuren 2A - 2D, in der anstelle der als Teilchen ausgestalteten Partikel 50 der Partikelklasse 2 ein Protein 52 eingesetzt wird, das mit einer Spezifität zum Partikel 40 der Partikelklasse 2 ausgestattet ist. In diesem Fall handelt es sich um ein biotinyliertes Protein 52, in diesem Falle BSA.

Eine weitere Variante stellt in den Figuren 4A-4D gezeigte Verfahrensweise dar, in der Antikörper 10 als Fängerantikörper auf einem Träger 15 immobilisiert sind. Ein Analyt 20 wird vom Fängerantikörper 10 gebunden, sodass der Immunkomplex 1 sich ausbilden kann. Anders als in Figur 2A beschrieben wird nicht ein weiterer Antikörper, der Beispielsweise mit Biotin 31 markiert werden kann und als Detektionsantikörper 30 wirkt, hinzugegeben, sondern es wird ein Partikel 40 der Partikelklasse 1 zugegeben, auf dessen Oberfläche zwei unterschiedliche Antikörper 45, 48 gebunden sind, wobei der erste Antikörper 45, der an den Partikel 40 der Partikelklasse 1 gebunden ist, an den Immunkomplex 1 bindet. Der Partikel 40 der Partikelklasse 1 weist darüber hinaus noch einen Antikörper 48 auf, der mit Oberflächenmarkierungen 55 auf dem Partikel 50 der zweiten Partikelklasse bindend in Wechselwirkung tritt. Dabei kann der Antikörper 48 beispielsweise gegen FITC 55 gerichtet sein, wohingegen der Antikörper 45 gegen den Analyten 20 nicht an die gleiche Bindungsstelle wie der Fängerantikörper 10 bindet. Nach Ausbildung des in Figur 4C gezeigten Multipartikelkomplexes kann dann in einem weiteren Schritt, wie in Figur 4D gezeigt, wiederum eine Vielzahl der Partikel 40 der Partikelklasse 1, wie in Figur 4B verdeutlicht, zugesetzt werden. Der Antikörper 48 bindet dann an freie Stellen 55 des Partikels 50 der zweiten Partikelklasse, wodurch sich eine weitere Verstärkung des Signals ergibt.

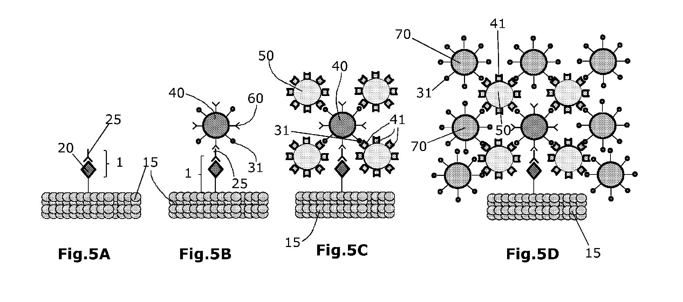

Figur 5 zeigt eine weitere ausführbare Variante unter Verwendung von Partikeln, die drei unterschiedlichen Klassen angehören, wobei zunächst gemäß Figur 5A auf einem Träger 15 ein immobilisiertes Antigen 20 präsentiert wird, an das ein Antikörper 25 aus der zu untersuchenden Probe bindet. Es hat sich diesmal also ein Immunkomplex 1 aus immobilisiertem Liganden 20 und Antikörper 20 aus der Probe gebildet.

Gemäß Figur 5B bindet an diesen Immunkomplex 1 aus Antikörper 25, Ligand 20, der auf den Träger 15 immobilisiert ist, ein Partikel 40 der Partikelklasse 1. Dieser trägt an seiner Oberfläche Antikörper 60 gegen die F_{c}-Region von IgG-Antikörpern, die mit dem Antikörper 25 des Immunkomplexes 1 reagieren und darüber hinaus, beispielsweise Biotin 31. Durch den Antikörper 60 vermittelt, bindet der Partikel 40 der Partikelklasse 1 an den am Träger 15 immobilisierten Immunkomplex 1. Die Biotinmarkierungen 31 dienen zur Bindung der Partikel 50 der Partikelklasse 2, die in einem nächsten Schritt zugesetzt werden (siehe Figur 5C). Die bereits vorher beschriebenen Partikel 50 der Partikelklasse 2 tragen an ihrer Oberfläche Avidinmoleküle, die mit den Biotinmolekülen 31 der Partikel 40 der Partikelklasse 1 eine Bindung eingehen und anhaftend ein Netz von Partikeln ausbilden. Gemäß Figur 5D kann zur weiteren Verstärkung des Signals dann eine Partikelklasse 70 zugefügt werden, an deren Oberfläche wiederum Biotinmolküle 31 anhaften und mit freien Avidinmolekülen auf der Oberfläche der Partikel 50 in Wechselwirkung treten.

Gemäß Figur 5D ist die enorme Verstärkung des Signals unmittelbar erkennbar. Dieses Ausführungsbespiel kann eingesetzt werden bei serologischen Tests, in dem entsprechend indikative Antikörper nachgewiesen werden müssen.

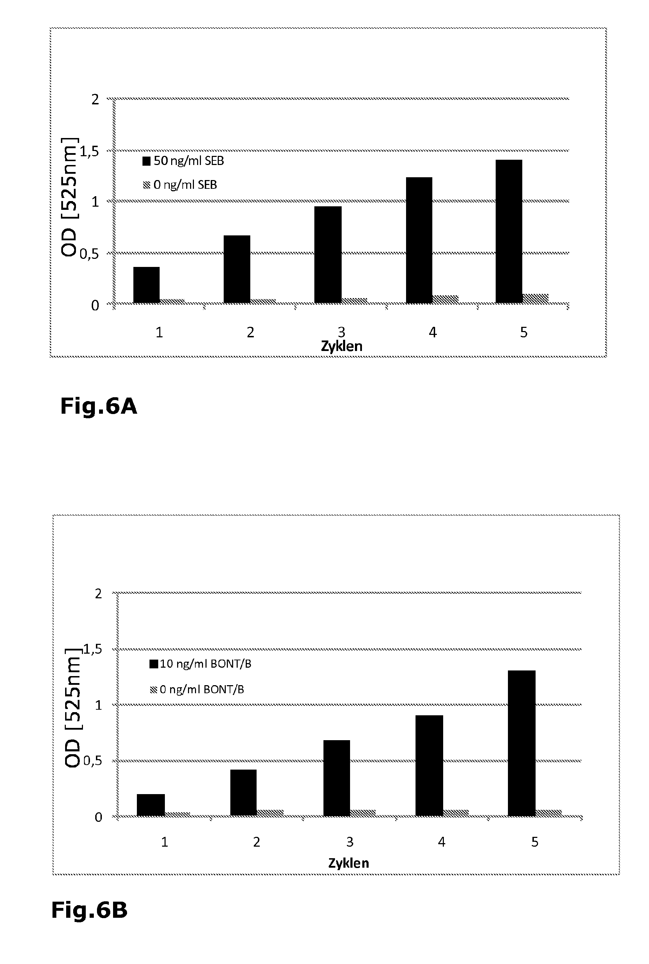

Die Figuren 6 A - C betreffen die grafische Wiedergabe der Tabellenergebnisse der jeweiligen Beispiele 1-3.

### Beispiel 1

### Detektion von Staphylokokken Enterotoxin B (SEB)

In dem nachstehenden Verfahren wird beschrieben wie man eine spezifische Signalverstärkung auf einer immunologisch funktionalisierten Oberfläche erzielen kann. Als Grundlage hierfür dient eine auf PE basierende 3 dimensionale zylinderförmige gesinterte Matrix ("Fritte") auf deren Oberfläche Antikörper immobilisiert sind. Diese Antikörper binden spezifisch aus einer Lösung SEB (Staphylokokken Enterotoxin B). Anschließend werden zu diesem Immunkomplex Partikel der Klasse 1 zugegeben. Partikel der Klasse 1 besitzen eine Spezifität (Antikörper) gegen SEB und binden somit an den Immunkomplex. Darüber hinaus besitzen sie auch eine Spezifität gegen Haptene die auf Partikel der Klasse 2 immobilisiert sind. Somit binden in einem Folgeschritt Partikel der Klasse 2 an Partikel der Klasse 1. Es entsteht ein oligomerer Partikelkomplex. Dieser wird durch wechselseitige Zugabe (Cycling) beider Partikelklassen vergrößert. Beide Partikelklassen besitzen eine Eigenabsorbtion bei 525 nm. Diese kann photometrisch vermessen werden. Die optische Dichte wird mit zunehmenden Zyklenzahlen größer und erzeugt eine spezifische Färbung des Filters.

Durchführung:

| **Nr.** | **Arbeitsschritt** | **Volumen** | **Reagenz** | **Zeit** | **Messung OD [ ]** |
|---|---|---|---|---|---|
| 1 | Probenaufgabe | 750µl | Probe [50 ng/ml SEB bzw. 0 ng/ml SEB] | 6 Minuten | |
| 2 | Cycling | 500 µl | Partikel der Klasse 1 | 6 Minuten | |
| 3 | | 750µl | Waschpuffer | | ja |
| 4 | | 500 µl | Partikel der Klasse 2 | 6 Minuten | |
| 5 | | 750 µl | Waschpuffer | | optional |
| 6 | | | Wiederholung ab Schritt 2 | | |

| | | | | | |
|---|---|---|---|---|---|
| (1)750 µl der mit SEB versetzten Probe wird auf die Säule pipettiert und bindet dort spezifisch an dem immobilisierten Antikörper. Die Inkubationszeit von 6 Minuten wird abgewartet. (2)500 µl Partikel der Klasse 1 werden aufgegeben. Die Inkubationszeit von 6 Minuten wird abgewartet. (3) Mit 750 µl Waschpuffer wird überschüssiger Partikel herunter gewaschen. (4)500 µl Partikel der Klasse 2 wird aufgegeben und binden an Partikel der Klasse 1. Die Inkubationszeit von 6 Minuten wird abgewartet. (5) Mit 750 µl Waschpuffer wird überschüssiger Partikel wieder herunter gewaschen. (6) In Abhängigkeit der gewünschten Zyklenzahl wird mit Schritt 2 wieder begonnen. | | | | | |

### Ergebnisse

| | OD | | | | |
|---|---|---|---|---|---|
| | Zyklen | | | | |
| SEB [ng/ml] | 1 | 2 | 3 | 4 | 5 |
| 50 | 0,358 | 0,664 | 0,952 | 1,236 | 1,407 |
| 0 | 0,047 | 0,052 | 0,061 | 0,078 | 0,09 |

Siehe Figur 6A

### Beispiel 2

### Detektion von BoNT/B

In vergleichbarer Weise wurde das voran beschriebene Verfahren auf den Nachweis von BoNT/B angewendet.

### Ergebnisse

Tabelle BoNT-B mit spezifischen Antikörpern

| | OD | | | | |
|---|---|---|---|---|---|
| | Zyklen | | | | |
| BONT/B [ng/ml] | 1 | 2 | 3 | 4 | 5 |
| 10 | 0,2 | 0,413 | 0,68 | 0,9 | 1,3 |
| 0 | 0,04 | 0,055 | 0,06 | 0,063 | 0,064 |

Siehe Figur 6B

### Beispiel 3

### Detektion von Tetanus-IgG (Pferd) aus Serum nach Ausführungsbeispiel 3

In diesem Ausführungsbeispiel wird der Titer an Tetanus-IgG aus Pferdeserum bestimmt. Als Grundlage dient eine 3 dimensionale gesinterte Matrix aus Polyethylen. Auf deren Oberfläche ist Tetanus-Toxoid immobilisiert. Die Tetanus spezifischen IgG aus der Probe binden an das immobilisierte Antigen unter Ausbildung eines Immunkomplexes. An diesen Immunkomplex binden Partikel der Klasse 1 über ihre Spezifität gegen den Fc-Teil des gebundenen Pferde-IgG. Darüber hinaus besitzen die Partikel der Klasse 1 noch eine Biotin Markierung. Partikel der Klasse 2 besitzen eine Avidin Markierung und binden an die Biotin Markierung der Partikel der Klasse 1. Es kommt zur Ausbildung eines Multipartikelaggregates. Dieses kann photometrisch gemessen werden. Zur Verstärkung dieses Aggregates werden Partikel der Klasse 3 hinzu gegeben. Diese Partikel besitzen eine Biotin Markierung und binden an das Avidin der Partikel der Klasse 2. Durch wechselseitige Aufgabe von Partikel der Klasse 2 und Partikel der Klasse 3 entsteht ein spezifisches oligomeres Partikelaggregat, welches photometrisch gemessen wird. Hierbei korreliert die Intensität des gemessen Signals mit dem Tetanus-IgG Titer aus dem Pferdeserum.

### Durchführung

| **Nr.** | **Arbeitsschritt** | **Volumen** | **Reagenz** | **Zeit** | **Messung OD [ ]** |
|---|---|---|---|---|---|
| 1 | Probenaufgabe | 750 µl | Probe [0,01 IE/ml Teta-nus-IgG bzw. 0 IE/ml Tetanus-IgG] | 6 Minuten | |
| 2 | initiale Detektion | 500 µl | Partikel der Klasse 1 | 6 Minuten | |
| 3 | waschen | 750 µl | Waschpuffer | | |
| 4 | Cycling | 500 µl | Partikel der Klasse 2 | 6 Minuten | |
| 5 | | 750 µl | Waschpuffer | | ja |
| 6 | | 500 µl | Partikel der Klasse 3 | 6 Minuten | |
| 7 | | 750 µl | Waschpuffer | | optional |
| 8 | | | Wiederholung ab Schritt 4 | | |

| | | | | | |
|---|---|---|---|---|---|
| (1) 750 µl Pferdeserum werden aufgegeben. Tetanus-IgG binden spezifisch an immobilisiertes Tetanus-Toxoid. (2) 500 µl Partikel der Klasse 1 werden aufgegeben und binden spezifisch an gebundenes Tetanus-IgG. (3) 750 µl Waschpuffer werden aufgegeben um überschüssigen Partikel der Klasse 1 zu entfernen. (4) 500 µl Partikel der Klasse 2 werden aufgegeben und binden an Partikel der Klasse 1. (5) 750 µl Waschpuffer werden aufgegeben um überschüssigen Partikel der Klasse 2 zu entfernen. Die optische Dichte [OD 525nm]wird gemessen. (6) 500 µl Partikel der Klasse 3 werden aufgegeben und binden an Partikel der Klasse 2. (7) 750 µl Waschpuffer werden aufgegeben um überschüssigen Partikel der Klasse 3 zu entfernen. (8) Je nach gewünschtem Verstärkungsgrad werden die Schritte ab Nr. 4 wiederholt (Cycling) | | | | | |

### Ergebnisse

| | OD | | | | |
|---|---|---|---|---|---|
| | Zyklen | | | | |
| Tetanus-IgG Pferd [IE/ml] | 1 | 2 | 3 | 4 | 5 |
| 0,01 | 0,13 | 0,26 | 0,58 | 0,92 | 1,26 |
| 0 | 0,03 | 0,036 | 0,04 | 0,048 | 0,054 |

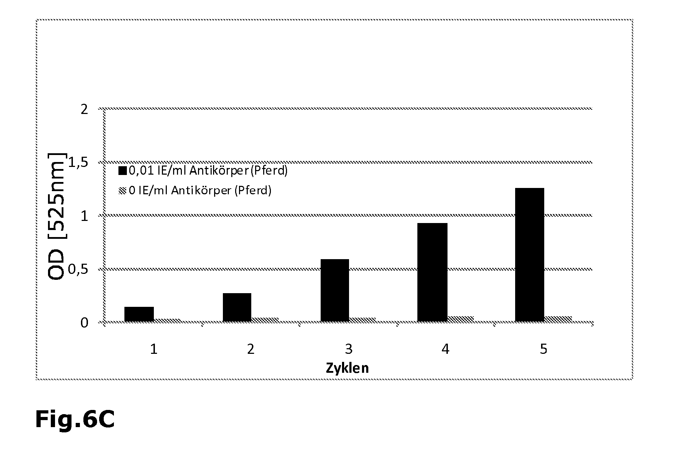

Siehe Figur 6C

## Patentansprüche

1. Verfahren zur Verstärkung von Signalen in heterogenen Bindungsassays auf Basis eines Donator/Rezeptorkomplexes (1) aus einem Rezeptormolekül (10) und einem Donatormolekül (20), das vom Rezeptormolekül (10) gebunden wird wobei
- in einem ersten Schritt der Donator/Rezeptorkomplex (1), gebildet wird,
- danach in einem zweiten Schritt mindestens ein Partikel einer ersten Partikelklasse zu dem gebildeten Donator/Rezeptorkomplex (1) gegeben wird,
- der mindestens eine Partikel der ersten Partikelklasse an den Donator/Rezeptorkomplex (1) koppelt und
- der mindestens eine an den Donator/Rezeptorkomplex (1) gekoppelte Partikel der ersten Partikelklasse n ≥ 2, wobei n eine natürliche Zahl ist, weitere Bindungsstelle aufweist, an die mindestens ein Partikel einer zweiten Partikelklasse koppeln kann,
- der Partikel der zweiten Partikelklasse seinerseits mindestens m ≥ 2, wobei m eine natürliche Zahl ist, Bindungsstellen aufweist an die mindestens ein Partikel der ersten Partikelklasse koppeln kann,
- in einem dritten Schritt mindestens ein Partikel der zweiten Partikelklasse zugegeben wird und
- gegebenenfalls die Schritte, insbesondere der zweite und dritte Schritt, mindestens einmal wiederholt werden.

2. Verfahren nach Anspruch 1, wobei nach der Zugabe des mindestens einen Partikels der zweiten Partikelklasse im dritten Schritt des Anspruchs 1 mindestens ein Partikel einer dritten, von den Partikeln der ersten Partikelklasse unterschiedlichen Partikelklasse zugegeben wird.

3. Verfahren nach Anspruch 1 oder 2, wobei n und m jeweils > 2 ist, insbesondere 3, 4, 5.

4. Verfahren nach mindestens einem der vorangehenden Ansprüchen, wobei die Partikel der ersten und zweiten Partikelklasse ungefähr die gleiche Größe oder unterschiedliche Größe aufweisen.

5. Verfahren nach mindestens einem der vorangehenden Ansprüchen, wobei die Partikelgröße eine mittlere Größe von 5 nm bis 5 µm aufweisen.

6. Verfahren nach mindestens einem der vorangehenden Ansprüche, wobei als Partikel der Partikelklasse organische oder anorganische Partikel oder Kombinationen davon eingesetzt werden.

7. Verfahren nach Anspruch 5, wobei als anorganische Partikel Goldpartikel magnetische Partikel, oder als organische Partikel Partikel aus Polymeren und Biopolymeren, wie Latex, Proteine, insbesondere fluoreszierende Proteine wie Phycoerythrin, Phycocyanin, oder Zellulosederivate, Dextranderivate eingesetzt werden.

8. Verfahren nach mindestens einem der Ansprüche 3 bis 6, wobei Goldpartikel mit einem mittleren Durchmesser von 5-50 nm, insbesondere 10-20 nm, Latexpartikel mit Farbstoff oder Fluoreszenzfarbstoff in einer Polymermatrix mit einem mittleren Durchmesser von 10 nm-4 µm, insbesondere 100 nm-400 nm, Farbstoffdispersionen mit Partikeln mit einem mittleren Durchmesser von 100 nm-500 nm, Magnetpartikel mit einem mittleren Durchmesser von 10 nm-4 µm, insbesondere 40 nm-400 nm oder Polymere, insbesondere Biopolymere wie Proteine insbesondere fluoreszierende Proteine wie Phycoerythrin, Phycocyanin, oder Zellulosederivate, Dextranderivate eingesetzt werden.

9. Verfahren nach mindestens einem der vorangehenden Ansprüche, wobei die Partikel der ersten, zweiten und/oder dritten Partikelklasse detektierbar sind.

10. Verfahren nach mindestens einem der vorangehenden Ansprüchen" wobei die freien Bindungsstellen der ersten und zweiten Partikelklassen wechselseitig durch ein Ligand/Rezeptorpaar gebildet wird.

11. Verfahren nach Anspruch 10, wobei das wechselseitige Ligand/Rezeptorpaar gebildet wird durch Avidin/Streptavin, Protein A/ IgG, insbesondere der F_{c} Teil von IgG, Lektin/Oligosaccarid, Polymerbindungsdomaine/ Biopolymer, anti Hapten-Antikörper/Hapten oder Kombinationen davon.

12. Verfahren nach Anspruch 9, wobei die Detektion der detektierbaren Partikel durch spektroskopische Methoden, wie Durchlichtphotometrie, Reflektometrie, Fluoriometrie, Luminometrie, Bestimmung der Brechzahl und Doppelbrechung, durch bildgebende Verfahren, durch elektrische Methoden wie Konduktometrie, Potentiometrie, oder magntische Methoden wie lineare und nichtlineare Suszeptometrie und Relaxometrie auf Basis von Giant Magnetic Resistoren, Hallsensoren, Flux Gate Sensoren, Squids oder Induktionsspulen erfolgt.

13. Verfahren nach mindestens einem der vorangehenden Ansprüche, wobei der Donator/Rezeptorkomplex (1) ein Immunkomplex ist.
